# EUROPEAN PATENT SPECIFICATION

(11) **EP 3 332 707 B1**
(45) Date of publication and mention of the grant of the patent: **30.10.2019**
(21) Application number: 16202513.4
(22) Date of filing: 06.12.2016
(51) Int. Cl.: A61B 6/00

(54) **SUPPORT FOR THE ARC OF A MOBILE X-RAY DEVICE**
TRÄGER FÜR DEN BOGEN EINER MOBILEN RÖNTGENVORRICHTUNG
SUPPORT POUR L'ARC D'UN DISPOSITIF À RAYONS X MOBILE

(43) Date of publication of application: 13.06.2018
(73) Proprietor: AGFA NV, 2640 Mortsel (BE)
(72) Inventor: Kopp, Edgar-Gerald, 2640 Mortsel (BE)
(74) Representative: Verbrugghe, Anne Marie L.

(56) References cited:
- US-A1- 2006 245 539
- US-A1- 2010 278 300
- US-A1- 2012 148 031

## Description

### Technical Field

The present invention relates generally to the acquisition of cone beam images performed on a mobile X-ray device

### Background Art

Cone beam computed tomography (or CBCT, also referred to as C-arm CT) is a medical imaging technique consisting of X-ray computed tomography where the X-rays are divergent, forming a cone. CBCT has become increasingly important in treatment planning and diagnosis in implant dentistry, interventional radiology (IR), among other things. The advantage of CBCT over conventional CT is that the delivered dose to the patient is lower in comparison with a CT examination. But, there is also an economic reason, namely that the technique can in principle be performed using conventional C-arm based X-ray devices which are much less expensive in comparison with CT scanner equipment.

Perhaps because of the increased access to such technology, CBCT scanners are now finding many applications in dentistry, such as in the fields of oral surgery and orthodontics. CBCT techniques are typically implemented in dedicated X-ray equipment for this medical domain. Integrated CBCT is also an important tool for patient positioning and verification in image-guided radiation therapy (IGRT). In this application, the modality to perform the CBCT acquisition is the same as the radiotherapy treatment device itself.

Also other body parts (such as the extremities) are being imaged using CBCT techniques. Certain applications require that patients can be imaged in a standing or upright position. This is for instance the case in sports healthcare, but it is not limited to this domain. Various technical solutions exist to meet these requirements, and they can be divided into 2 groups:
- C-arm based systems (open ring)
- Ring-shaped systems (closed ring)

Both of these approaches have their particular limitations and constraints.

Most of the closed ring-shaped systems are application specific setups which vary between large stationary setups, and smaller but mobile devices. The large stationary devices referred to here are mostly integrated into a dedicated room, and cannot be used to image patients in a standing position, because they are used in combination with a table on which the patient is positioned during the acquisition. The rotation axis around which the source and detector pair revolves during acquisition is in this case horizontally oriented.

The smaller and mobile devices available on the market can in some occasions be used with standing patients, as they are often designed with a specific body part in mind (such as extremities). The application range for these devices is however limited. The patient access to the acquisition area and the manoeuvring space during the acquisition of these smaller devices is very limited, which reduces the patient comfort. These types of systems do mostly not allow use for other non-intended applications.

Nevertheless, systems are continuously being improved in order to overcome these limitations. Systems have been described in the art (such as for instance in US8348506) where a closed ring setup is disclosed which is especially adapted to facilitate the access for a patient to the acquisition center of the device with the intention to improve the patient experience in preparation of and during the acquisition.

The second type of CBCT systems are the so-called C-arm based systems, which are based on a C-shaped rotatable arm to which a digital X-ray detector and a X-ray collimator and source are mounted in opposing positions. In a single orbit about the patient, a complete volumetric dataset covering a large anatomic region of interest is generated from which submillimeter isotropic reconstructions can be created. The high efficiency two-dimensional X-ray detector allows excellent low-dose detectability, a capability not present on image intensifier detector-based 3D-angiographic systems.

C-arm based CBCT systems are typically integrated in either fixed or mobile X-ray systems.

The C-arm in a fixed X-ray system is typically mounted to the wall or ceiling of a dedicated room so that the detector-source pair that is fixed to said rigid C-arm can be rotated around a patient who is positioned on a horizontal table. The table is part of the x-ray room setup and is dedicated to the x-ray system. US-A1-2010/278300 discloses provision of a c-shaped support for a fixed CBCT system.

In a mobile x-ray system, the C-arm is mounted on a mobile cart that also comprises the x-ray generator, batteries and image processing workstation. The C-arm is mounted at a suspension point to the suspension system or C-arm stand. The entire mobile x-ray device can be moved to the room or area where the patient is located. The C-arm can be positioned around the patient who is typically lying in a bed or on an intervention table during the x-ray examination.

During CBCT acquisition, the C-arm is then typically oriented in upright (vertical) position, in order for the arc of the C-arm to perform a vertically or nearly vertically oriented circular path. A C-arm CBCT system generates a complete volumetric dataset via a single 210-220° (180° plus fan angle) rotation of the arc on which the detector-source pair is mounted. The patient is stationary during the entire acquisition. The inclination of the circular path for said rotation can be adapted slightly in order to accommodate for the patient position or particular acquisition requirements, and this is achieved by changing the inclination of the suspension point between the arc and the suspension system or C-arm stand.

Nevertheless, this inclination is adaptable only within certain limits. When the CBCT acquisition path of the detector-source pair describes a vertically oriented circular movement, acceptable forces (i.e. limited torques) are applied to the suspension point where the C-arm is attached to the C-arm suspension system. This is no longer the case when the circular acquisition path becomes more and more tilted, i.e. when the inclination of the arc of the C-arm enlarges. Especially in the most extreme case when the C-arm is oriented in a horizontal position, extreme torques are applied to the suspension point of the C-arm system when the detector-source pair rotates during the acquisition. Considering that a typical C-arm measures 1 meter in diameter, and the weight of a collimator and x-ray source combination accounts for around 100kg, the torque can result in such a configuration in 1000Nm. These extreme forces prevent a stable movement of the C-arm during the circular movement, as the combined weight of the arc, detector and source shift from one side to the other during their rotation. Apart from the fact that the internal friction of the arc and the suspension point may prevent the system to move at all under these circumstances, mechanical breakage under these circumstances is very likely. Therefore, in most C-arm systems, the tilt or inclination of the arc of the C-arm at the suspension point is electronically or mechanically limited to prevent mechanical damage and dangerous situations. So it can be safely stated that today no fixed neither mobile C-arm systems support CBCT acquisitions when the C-arm is in a horizontal position.

Another problem is the mechanical flex of the C-arm during the acquisition as a consequence of the weight and movement of the components. Reconstruction algorithms require at least the accurate notion of the positions of the source, detector and object during the acquisition. This accurate notion may be jeopardized in case that the mechanical setup is subject to mechanical forces. The magnitude of C-arm flex is several millimeters in the tube and detector components and increases when the system is more angulated (an example known to us showed up to 14mm of C-arm flex in projection space at 45°). In the most extreme case, where the C-arm is positioned horizontally (at 90°, or in the position which is required to be able to perform CBCT acquisitions on upright standing subjects) the described flex will be even more dramatic, and will prevent accurate image reconstruction in the case that no compensating measures are taken.

However, because the C-arm flex is reproducible, even at steep angulations, as long as appropriate geometric calibrations are performed, precise 3D reconstructions are possible. Nevertheless since the calibrations are cumbersome and complex to perform and since they need to be performed on a regular basis, it would be more practical if this could be at all avoided.

A third problem encountered during CBCT acquisition using a C-arm based system are the vibrations of the entire system (and consequently of the attached image generating components) during the circular movement of the arc. These vibrations cannot be ignored and have a negative impact on the resulting (reconstructed) image quality. It would be better if these vibrations could be reduced or compensated.

### Summary of invention

The present invention provides a positioning apparatus for supporting a rotable arc of a C-arm of a mobile x-ray apparatus during a CBCT acquisition, comprising a base configured for positioning said apparatus on a floor and to support a horizontally positioned C-shaped top surface, said base being adjustable in height, a horizontally positioned C-shaped top surface adapted to support said rotable arc, wherein said top surface comprises a friction reduction mechanism embedded in said top surface reducing the friction between said surface and said rotable arc in the direction of a horizontal circular trajectory performed by said arc during the CBCT acquisition, as set out in claim 1.

The above-described aspects are solved by the apparatus as set out in claim 1.

In the context of this invention, a mobile x-ray modality generally consists of two main components; the X-ray generator and image system on a portable imaging system (comprising the C-arm) and the workstation unit used to store and manipulate the images. The imaging system unit can perform a variety of movements that allow for use in a variety of conditions and even in interventional procedures such as cardiology, orthopaedics and urology.

The C-arm provides the appropriate structure to mount an image intensifier or digital flat panel detector and an X-ray tube with a beam limiting device (collimator) positioned directly opposite from each other. In the context of this invention, the C-arm consists of a suspension system or C-arm stand, a suspension point for the rotable arc, and the rotable arc itself. The inner C-arm is the C-shaped rigid arch on which the detector and source are mounted directly. In the context of this invention, the inner C-arm is called the rotable arc. The rotable arc is captured and held (at least partially) in an enveloping structure which is called the outer C-arm. This outer C-arm is pivotally attached to a (mostly vertically oriented) stand by means of a pivoting mounting structure. This pivoting mounting structure can be moved up or down on the stand.

The outer C-arm serves as a crawler carriage which is intended to capture and propagate the inner C-arm along a circular trajectory which spans typically 210-220 degrees in total.

The C-arm is capable of many movements. When the C-arm is positioned in it's upright position (the x-ray source and collimator oriented upwards, and the detector bottom wards), the following movements are supported by a typical device (the figures are measured on an actual device and serve as an example only):
- Horizontal travel: about 200 mm
- Orbital travel: about 115 degrees
- Motorized vertical travel: 460 mm
- C-arm rotation about the horizontal axis +/- 210 degrees

In the context of this invention, this latter C-arm rotation is called the horizontal circular trajectory of the C-arm presuming that the C-arm structure is rotated into the horizontal position, which is the position suitable for a CBCT acquisition of an upright or standing subject. This movement is effectuated on the inner C-arm by the motor in the crawler carriage of the outer C-arm structure. In the context of this invention, the inner C-arm performs the horizontal circular trajectory which spans a maximum of 210 degrees during the CBCT acquisition. The source-detector pair travels over an arch of about 210 degrees (180 degrees plus fan angle) in order to collect the required projection data for a cone beam image reconstruction.

The invention applies to mobile x-ray devices, of which the mobile aspect relates to the feature that the entire modality can be moved from one location to the other. In other words, the system including the C-arm is mobile and can be easily moved and repositioned at one location.

The docking mechanism of the present invention is a two-part mechanical disposition of which one part is implemented on the mobile X-ray modality and the other on the support and positioning apparatus of this invention. More particularly and preferably, there is a locking mechanism foreseen at the modality side which can clamp the mobile x-ray system to especially foreseen points at the support and positioning apparatus side. The docking mechanism ensures that the mobile x-ray modality can be firmly but removably attached to the positioning apparatus, and this at precisely predetermined clamping points, in order to ensure a predetermined and firm coupling of both devices.

Specific examples and preferred embodiments are set out in the dependent claims.

The present invention is beneficial in that the support apparatus as disclosed in this application enables the use of a standard mobile x-ray device (C-arm) to perform CBCT acquisitions with horizontal C-arm movement.

The present invention overcomes the mechanical constraints of a standard mobile x-ray device to ensure a stable and yet smooth movement of the C-arm during the circular movement for a CBCT acquisition. A further advantage of the disclosed system is that the mobile x-ray device can easily be mounted and dismounted from the supporting apparatus by means of the docking mechanism. This ensures that the mobile x-ray device can still be used as before. Another advantage is that no elaborate calibrations or measurements need to be performed to account for the mechanical deformations of the acquisition geometry during horizontal positioning of the arc. Also, vibrations of the entire system which are typically induced by the movement of the arc during the acquisition cycle can be prevented.

Further advantages and embodiments of the present invention will become apparent from the following description and drawings.

### Brief description of drawings

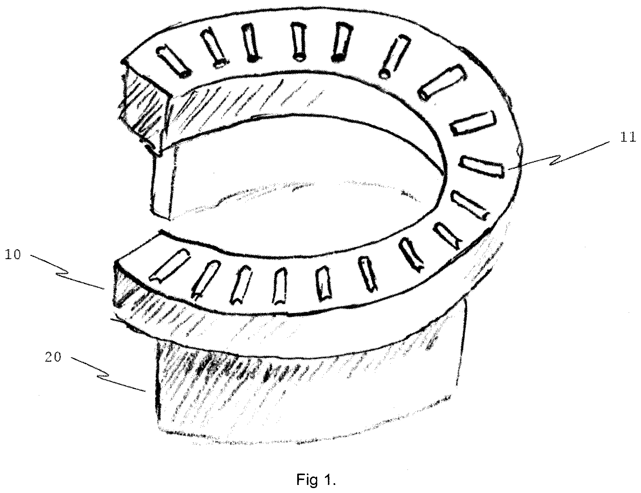

Fig 1. shows an embodiment of a positioning apparatus of the invention. The drawing shows the embodiment in a perspective view, and indicates its parts: a horizontally positioned C-shaped top surface [10], a base [20] and an embodiment of a friction reduction mechanism [11].

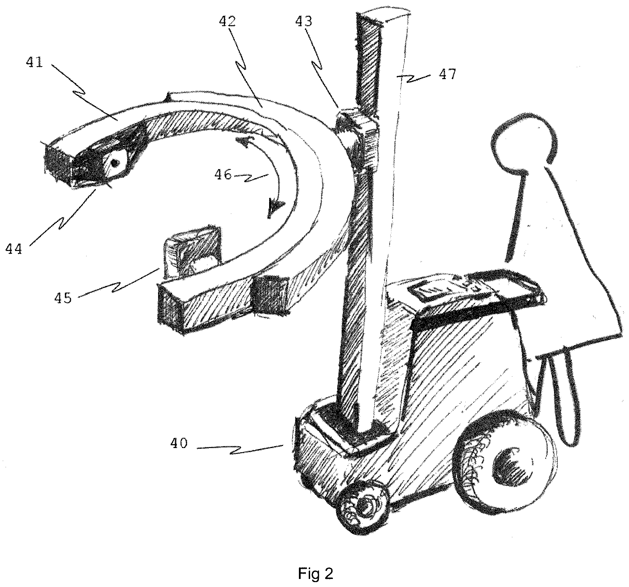

Fig 2. Shows a mobile C-arm based x-ray device of which the C-arm structure is rotated into the horizontal position. The main components of the C-arm structure and the x-ray device are shown and indicated on the drawing; the mobile cart [40], inner C-arm structure [41], outer C-arm structure [42], arrow indicating the direction of the horizontal circular trajectory of the inner C-arm [46] during a CBCT acquisition. An x-ray source [44] and detector [45] pair is mounted in opposing position from each other on the inner C-arm structure [41]. The C-arm structure is mounted by means of the pivoting mounting structure [43] to the vertical stand [47]. The pivoting mounting structure can move up and down.

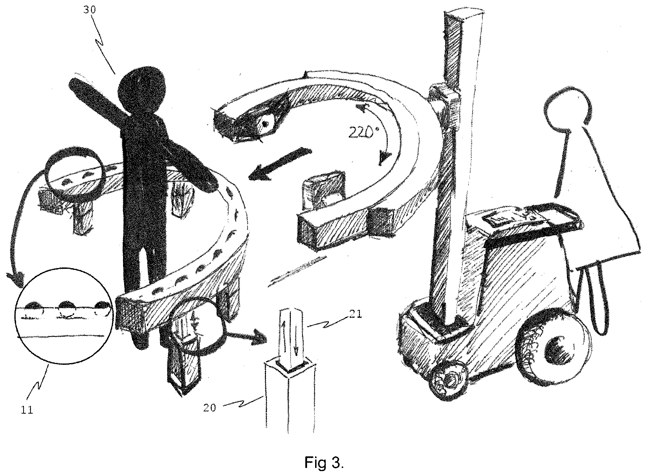

Fig 3. shows a slightly different embodiment of the positioning apparatus in combination with a mobile C-arm based x-ray device, and an upright standing patient [30] positioned in the acquisition center of the device. The difference shown in this embodiment of the device (in comparison with the one depicted in Fig 1.) is its base [20] which comprises four supporting legs and which are adjustable in height [21]. Also a different type of a friction reduction mechanism [11] is shown; a set of wheels aligns to the rotation path of the inner C-arm structure.

### Description of embodiments

In the following detailed description, reference is made in sufficient detail to the above referenced drawings, allowing those skilled in the art to practice the embodiments explained below.

Embodiments of the present invention provide a positioning apparatus for supporting a rotable arc [41] of a C-arm of a mobile x-ray apparatus during a CBCT acquisition, comprising a base [20] configured for positioning said apparatus on a floor and to support a horizontally positioned C-shaped top surface [10], a horizontally positioned C-shaped top surface adapted to support said rotable arc, wherein said top surface comprises a friction reduction mechanism [11] embedded in said top surface reducing the friction between said surface and said rotable arc [41] in the direction of a horizontal circular trajectory performed during the CBCT acquisition.

The base of the apparatus in this invention is referred to in Fig. 1 as [20] is the part of the apparatus which serves as a pedestal for the apparatus on the floor. Since the apparatus is intended to carry the weight of at least the arc of the C-arm [41] and [42], the detector [45] and the x-ray source [44] with the collimator, said base needs to sufficiently strong to carry said weight. Therefore the base [20] can be carried out in different possible embodiments as differently shaped floor standing solutions, be it a solid base, or a base comprising supporting legs (such as the legs of a table). Stability is the most important factor, and since the weight to be carried can easily exceed 200kg, the materials will have to be selected accordingly.

The base of the apparatus can in some cases be anchored to the floor so as to make the installation more permanent and at the same time provide more stability. An anchored base has the advantage that the mechanical stability of the entire CBCT x-ray system will increase which will prevent mechanical deformations, vibrations and interferences alike which can have a negative impact on the acquisition quality.

Optionally can the base of the apparatus be adapted to be adjustable in height [21]. Regardless of the type of base which is implemented, the provision for adjusting the height of the base is to ensure that the entire surface top [10] is adjustable in height.

Since the base of the apparatus has to support the C-shaped arc of the C-arm, the shape of the base is adapted to the shape of the arc itself. This means that the shape of the solid pedestal, or in the case that a set of legs are used, that the arrangement of the at least 3 supporting legs is adapted to sustain the C-shaped arc.

The shape of the C-shape is nearly circular, meaning that there is a cut-out foreseen in the otherwise circular structure to allow the patient to enter the nearly circular shaped acquisition center. The cut-out covers at least 30° leaving a minimal opening for an accessible entry to the centre of the apparatus, while said cut-out should be at most 140°-150° in order to be able to support the circular motion of the C-arm (of typically 210°-220°). The latter adaptation results in a shape of the apparatus which more approaches a closed "C" in comparison with the shape of the supported arc itself.

Similarly is the horizontally positioned C-shaped top surface of the apparatus adapted to the shape of the arc of the C-arm it needs to support. The ideal shape of the top surface of the positioning apparatus is preferably an exact fit of the flat area of the surface of the underside of the horizontally oriented rotatable arc. This flat area may cover the entire horizontal underside of said arc, or could be more narrow area of the arc's surface only.

The horizontally positioned C-shaped top surface rests on the base of the apparatus as described above. This top surface typically will have to made of a strong material since it has to distribute the weight of the support C-arm over its surface. The top surface thus will have a certain thickness at least determined by the weight it needs to distribute.

The top surface not only has to support the weight of the supported arc of the C-arm, it also needs to facilitate the circular motion of the arc during its movement along the shape of the C-shaped surface top. In other words, the top surface should be adapted as such that the friction between the arc and the top surface of the positioning apparatus (on which the arc rests) is minimized during their respective (circular) motion. This can be achieved by means of different implementations of friction reducing mechanisms or materials. In this context, the friction between the 2 gliding surfaces could be reduced using a combination of 2 low friction materials known in the art for use as the contact surfaces for the two parts. Examples of such materials are polytetrafluoroethylene (PTFE) (better known as teflon) or" Delrin" (a product from DuPont). Lubrication of the surfaces seems to be impractical to implement for this type of application.

Alternatively, the friction between the 2 parts (arc of the C-arm and surface top of the positioning apparatus) could be reduced by applying mechanical adaptations to the surface top. These adaptations can be a series of wheels which are applied to any of the 2 surfaces (top surface of the positioning apparatus, or lower surface of the arc of the C-arm). The travel direction of said arrangement of wheels then follows the same circular trajectory of the relative movement of the arc with respect to the positioning apparatus. The rotation axes of the wheels should thus be arranged in radial direction from the center of the C-shape and parallel to the surface top. A minimum of 3 wheels has to be applied in order to ensure minimal contact, but it is preferable to have a multitude of wheels (more then 20) arranged at equidistant positions over the entire length of the C-shape, and this even in more then 1 circular row. More wheels ensure a better distribution of the weight of the arc over the available wheels, and also ensures a smoother (vibration free) circular movement.

In another embodiment, the arrangement of wheels may be replaced with a set of bearings, ball-bearings or roller-bearings built into the surface of the surface top. The rolling direction of these alternatives should follow the same circular shape of the movable part of the C-arm (the arc) in order to optimally reduce the friction. The wheels, bearings, ball-bearings or roller-bearings are thus setup in an arc-shaped configuration wherein the rolling direction follows the circular shape of the movable part of the C-arm.

The positioning apparatus in this invention is always used in combination with a mobile C-arm based x-ray device, which has the capability of performing cone beam acquisitions. The mobile x-ray device has to fulfil the following requirements:
- it is capable of orienting the C-arm into a horizontal position,
- it has to be able to execute an CBCT acquisition cycle (perform a continuous exposure during a semi-circular acquisition path over an arc of about 210-220°)
- it has to be able to process/reconstruct the acquired image data.

In a first step, and as a preparatory step for positioning the C-arm onto the positioning apparatus, the C-arm is positioned into a vertical (or upright) position, making sure that the suspension point is elevated higher then the surface top of the positioning apparatus. Subsequently, the arc of the C-arm is rotated into its rest position (i.e. the source will be on top of the C-arm and the detector at the bottom). When the C-arm is in this position, the C-arm can be tilted (rotation around the suspension point) without any risk for damaging the C-arm into a horizontal position. Putting the arc first in its rest position ensures that the arc is more or less into equilibrium, avoiding unnecessary forces (torque) on the suspension point.

In a seconds step, the mobile x-ray device is approached to the positioning apparatus. The C-arm (which is in a horizontal position) is moved over and aligned with the surface top, before it is lowered onto it.

The height of the surface top of the positioning apparatus determines the range of imaging applications which can be performed on the standing subject. In case that the positioning apparatus can support the arc of a C-arm at different adjustable heights, a wide range of applications can be supported. When the height can be adjusted from 0 to 100cm, almost any region of interest of lower part of the human body can be imaged on an upright standing or seated subject, which is a huge advantage over the applicability of the different devices known in the art.

When the arc of the C-arm is lowered onto the surface top of the apparatus, it has to be ensured that the arc is correctly and firmly positioned on the surface allowing the arc to perform the circular movement along the path foreseen by the friction reducing mechanism.

In another embodiment of this invention, an optional docking mechanism can be foreseen which requires adaptations to both the positioning apparatus and an immobile part during the acquisition sequence of the C-arm (such as the outer C-arm). An immobile part, being a part of the C-arm which does not move during the movement required for a CBCT acquisition. The docking mechanism has to be understood as that the positioning apparatus is able to receive a part of the C-arm in order to lock it into a mechanical grip to ensure a more correct and stable alignment of the C-arm arc with the surface top. Many different docking mechanism implementations can be envisaged; but a common requirement is that docking mechanism is able to immobilize both components in the 3 orthogonal directions. The docking mechanism therefore can be assumed to make contact with at least 2 points of both components, in order to assure firm attachment.

As soon as the C-arm is positioned (and optionally docked) onto the positioning apparatus, the patient can be positioned and the x-ray device can start the cone beam acquisition. For that purpose, the arc will be rotated to one of its extreme angles to assume the starting point. The positioning apparatus sustains at this point at least 75% of the total surface (and weight) of the arc, which relieves the C-arm from the tremendous forces it would otherwise be applied to.

When the circular movement is initiated to acquire the CBCT dataset, the arc rests on the (almost) frictionless surface of the positioning apparatus, allowing a smooth and well controlled passage over the entire trajectory. The above mentioned negative effects (vibrations, mechanical deformations, ...) are completely absent during the acquisition, which results in a much better image reconstruction quality and less mechanical problems such as wear and breakage.

## Claims

1. A positioning apparatus for supporting a rotatable arc [41] of a C-arm of a mobile x-ray apparatus [40], the C-arm being mounted on a mobile cart with a stand and comprising an inner rotatable C-arm and a rigid outer C-arm which is attached to the stand by means of a pivoting mounting structure such that the C-arm can be oriented into a horizontal position said positioning apparatus comprising:
- a base [20] configured for positioning said apparatus on a floor and to support a horizontally positioned C-shaped top surface [10], the shape of the C-shaped top surface being an exact fit of the flat area of the surface of the underside of the horizontally oriented rotatable arc,
- a horizontally positioned C-shaped top surface [10] adapted to support said horizontally positioned rotable arc [41],
wherein said top surface [10] comprises:
- a friction reduction mechanism [11] embedded in said top surface reducing the friction between said surface [10] and said rotable arc [41] in the direction of a horizontal circular trajectory [46] performed by said arc during the CBCT acquisition.

2. The apparatus of claim 1, wherein the C-shape is nearly circular.

3. The apparatus of claim 2, wherein a cut-out in the C-shape covers between 30° and 150°.

4. The apparatus of any of the previous claims, wherein said base is adjustable in height.

5. The apparatus of any of the previous claims comprising a docking mechanism for locking an immobile part of the horizontally positioned C-arm of the mobile x-ray device during a CBCT acquisition sequence in a fixed position to said base.

6. The apparatus of claim 5, wherein the docking mechanism attaches preferably to the pivoting point of the C-arm.

7. The apparatus of any of the previous claims, wherein said mechanical friction reduction mechanism [11] comprises a set of multiple wheels, balls, bearings or rollers built-in to the surface and setup in an arc-shaped configuration wherein the rolling direction of said wheels follows the circular shape of the movable part of the C-arm of the mobile x-ray device.

8. The apparatus of any of the previous claims, wherein the base comprises a single massive support structure.

9. The apparatus of any of the previous claims, wherein the base comprises at least 3 supporting legs.

10. An X-ray device for performing a CBCT acquisition of an upright standing patient, comprising:
- the apparatus of claim 1,
- a mobile X-ray device with a C-arm gantry supporting an X-ray source and digital detector positioned in a diametrically opposing configuration, and allowing a circular movement around the object to be imaged.

## Patentansprüche

1. Eine Positioniervorrichtung zum Tragen eines drehbaren Bogens [41] eines C-Arms einer mobilen Röntgenvorrichtung [40], wobei der C-Arm auf einem mobilen, mit einem Ständer ausgestatteten Wagen montiert ist und einen drehbaren C-Innenarm und einen steifen C-Außenarm umfasst, wobei der steife C-Außenarm mittels einer schwenkbaren Montagestruktur derart am Ständer befestigt ist, dass der C-Arm in eine horizontale Position orientiert werden kann, wobei die Positioniervorrichtung Folgendes umfasst:
- einen Träger [20], der so konfiguriert ist, dass er die besagte Vorrichtung auf einen Boden positioniert und eine horizontal positionierte C-förmige Deckfläche [10] trägt, wobei die Form der C-förmigen Deckfläche passgenau auf den flachen Bereich der Oberfläche der Unterseite des horizontal orientierten drehbaren Bogens abgestimmt ist,
- eine horizontal positionierte C-förmige Deckfläche [10], die so angepasst ist, dass sie den horizontal positionierten drehbaren Bogen [41] trägt,
wobei die Deckfläche [10] Folgendes umfasst:
- einen reibungsverringernden Mechanismus [11], die in die Deckfläche eingebettet ist, um die Reibung zwischen der Oberfläche [10] und dem drehbaren Bogen [41] in Richtung einer während der CBCT-Erfassung vom Bogen zurückgelegten horizontalen kreisförmigen Strecke [46] zu verringern.

2. Vorrichtung nach Anspruch 1, wobei die C-Form nahezu kreisförmig ist.

3. Vorrichtung nach Anspruch 2, wobei ein Ausschnitt in der C-Form zwischen 30° und 150° abdeckt.

4. Vorrichtung nach einem der vorstehenden Ansprüche, wobei der Träger höhenverstellbar ist.

5. Vorrichtung nach einem der vorstehenden Ansprüche, umfassend einen Andockmechanismus, mit dem ein immobiler Teil des horizontal positionierten C-Arms der mobilen Röntgenvorrichtung während einer CBCT-Erfassungssequenz in eine feste Position am Träger verriegelt wird.

6. Vorrichtung nach Anspruch 5, wobei der Andockmechanismus bevorzugt am Schwenkpunkt des C-Arms befestigt ist.

7. Vorrichtung nach einem der vorstehenden Ansprüche, wobei der die mechanische Reibung verringernde Mechanismus [11] einen Satz mehrfacher Räder, Kugeln, Lager oder Walzen umfasst, die auf der Oberfläche eingebaut sind und in einer bogenförmigen Konfiguration angeordnet sind, wobei die Rollrichtung der Räder der kreisförmigen Form des beweglichen Teils des C-Arms der mobilen Röntgenvorrichtung folgt.

8. Vorrichtung nach einem der vorstehenden Ansprüche, wobei der Träger eine einzelne massive Trägerstruktur umfasst.

9. Vorrichtung nach einem der vorstehenden Ansprüche, wobei der Träger mindestens 3 Stützbeine umfasst.

10. Eine Röntgenvorrichtung zum Ausführen einer CBCT-Erfassung eines aufrecht stehenden Patienten, umfassend:
- die Vorrichtung nach Anspruch 1,
- eine mobile Röntgenvorrichtung mit einer C-Armtraverse, die eine Röntgenquelle und einen digitalen Detektor trägt, die in einer diametral gegenüberliegenden Konfiguration positioniert sind und eine kreisförmige Bewegung um den zu bebildernden Gegenstand herum sichern.

## Revendications

1. Dispositif de positionnement servant à supporter un arc mobile [41] d'un bras en C d'un dispositif à rayons X mobile [40], ledit bras en C étant monté sur un chariot mobile pourvu d'un montant et comprenant un bras en C intérieur rotatif et un bras en C extérieur rigide, ledit bras en C extérieur rigide étant attaché au montant à l'aide d'une structure de montage pivotante de façon à ce que le bras en C puisse être orienté dans une position horizontale, ledit dispositif de positionnement comprenant:
- une base [20] configurée de façon à positionner ledit dispositif sur un sol et supporter une surface supérieure en C [10] horizontalement positionnée, la forme de la surface supérieure en C étant parfaitement adaptée à la zone plate de la surface du côté inférieur de l'arc rotatif horizontalement orienté,
- une surface supérieure en C [10] horizontalement positionnée adaptée de façon à supporter l'arc rotatif horizontalement positionné [41],
**caractérisé en ce que** ladite surface supérieure [10] comprend:
- un mécanisme réduisant la friction [11] encastré dans ladite surface supérieure et réduisant la friction entre la surface [10] et l'arc rotatif [41] dans le sens d'une trajectoire circulaire horizontale [46] parcourue par l'arc lors de l'acquisition CBCT.

2. Dispositif selon la revendication 1, **caractérisé en ce que** la forme en C est presque circulaire.

3. Dispositif selon la revendication 2, **caractérisé en ce qu'**une découpe dans la forme en C recouvre entre 30° et 150°.

4. Dispositif selon l'une quelconque des revendications précédentes, **caractérisé en ce que** la hauteur de la base est réglable.

5. Dispositif selon l'une quelconque des revendications précédentes, comprenant un mécanisme d'accueil permettant de verrouiller une partie immobile du bras en C horizontalement positionné du dispositif à rayons X mobile dans une position fixe à ladite base lors d'une séquence d'acquisition CBCT.

6. Dispositif selon la revendication 5, **caractérisé en ce que** le mécanisme d'accueil est attaché de préférence au point de pivotement du bras en C.

7. Dispositif selon l'une quelconque des revendications précédentes, **caractérisé en ce que** le mécanisme réduisant la friction mécanique [11] comprend un ensemble de multiples roues, billes, roulements ou rouleaux incorporés sur la surface et disposés dans une configuration sous forme d'arc, le sens de roulement des roues suivant la forme circulaire de la partie mobile du bras en C du dispositif à rayons X mobile.

8. Dispositif selon l'une quelconque des revendications précédentes, **caractérisé en ce que** la base comprend une seule structure de support massive.

9. Dispositif selon l'une quelconque des revendications précédentes, **caractérisé en ce que** la base comprend au moins 3 pieds de support.

10. Dispositif à rayons X pour exécuter une acquisition CBCT d'un patient en position debout, comprenant:
- le dispositif selon la revendication 1,
- un dispositif à rayons X mobile comprenant un portique de bras en C supportant une source de rayons X et un détecteur numérique positionnés dans une configuration diamétralement opposée et assurant un mouvement circulaire autour de l'objet à imager.
